# EUROPEAN PATENT APPLICATION

(11) **EP 4 239 055 A1**
(43) Date of publication of application: **06.09.2023**
(21) Application number: 21886777.8
(22) Date of filing: 27.10.2021
(51) Int. Cl.: C12N 1/20, A61K 35/74, A61P 37/04, A61P 35/00, A23L 33/135, C12R 1/01

(54) **APPLICATION OF PREVOTELLA STERCOREA STRAIN FOR CANCER PREVENTION OR TREATMENT**

(30) Priority: 27.10.2020 KR 20200140641; 26.10.2021 KR 20210143484
(71) Applicant: The Asan Foundation, Seoul 05505 (KR); University Of Ulsan Foundation For Industry Cooperation, Ulsan 44610 (KR); National Cancer Center, Goyang-si, Gyeonggi-do 10408 (KR)
(72) Inventor: PARK, Sook Ryun, Seoul 05649 (KR)
(74) Representative: Dehns
(86) International application number: PCT/KR2021/015146
(87) International publication number: WO 2022/092783

(57) **Abstract**

The present invention relates to the application of a *Prevotella stercorea* strain for cancer prevention or treatment and the like. The present inventors identified that a *Prevotella stercorea* strain increases the proliferation of T cells and increases IFN-γ secretion of T cells, and that *Prevotella stercorea* increases the anticancer efficacy of immune checkpoint inhibitors. Therefore, it is expected that a *Prevotella stercorea* strain or a culture solution thereof can be usefully used for enhancing immunity and increasing the anticancer effect of immune checkpoint inhibitors.

## Description

### [Technical Field]

The present invention relates to the application of a *Prevotella stercorea* strain for cancer prevention or treatment or the like.

The present application claims priority to and the benefit of Korean Patent Application Nos. 10-2020-0140641 and 10-2021-0143484 filed in the Korean Intellectual Property Office on October 27, 2020 and October 26, 2021, respectively, and all the contents disclosed in the specification and drawings of these applications are incorporated in the present application.

### [Background Art]

Cancer is one of the intractable diseases that mankind must overcome, and while huge amounts of capital are being invested in the development of cures for cancer, cancer has been the leading cause of death in Korea since the 1990s, with about 100,000 or more people being diagnosed with cancer and about 60,000 or more people dying from cancer each year. Cancer-inducing factors include smoking, ultraviolet rays, chemicals, food, stress, and other environmental factors, and not only is it difficult to develop therapeutic agents due to the variety of the cancer-inducing factors, but also the effects of therapeutic agents vary depending on the site of occurrence.

Anticancer therapies include removal of tumor tissue through surgery, radiation therapy, chemotherapy, and immune anticancer therapy. Anticancer agents used in anticancer therapy are applied differently for each cancer type. Chemical anticancer agents that are generally prescribed to cancer patients affect the survival mechanism of general normal cells including cancer cells, causing patients to experience side effects such as hair loss, fever, diarrhea, rashes, and weakened immunity. Thereafter, since targeted anticancer agents, which suppress singularities due to characteristic genetic mutations of cancer, have been developed based on genetic research on cancer, side effects caused by existing chemical anticancer agents have been significantly alleviated, but cancer cells that mutate very quickly can induce anticancer agent resistance in targeted anticancer agents, showing that there is a problem in that 100% of the sustained anticancer effects of targeted anticancer agents cannot be expected. Recently, since research on tumor microenvironments has been actively conducted, immunological anticancer drugs against various immune checkpoints that regulate patient immunity have been developed and used as therapeutic agents for patients. Among them, immunological anticancer agents that suppress PD-1/PD-L1 are known to show high therapeutic response in patients with melanoma, gastric cancer, lung cancer, and the like. Such immune anticancer agents have the function of suppressing the proliferation of cancer cells by activating the functions of immune cells within the tumor microenvironment. However, immune anticancer agents do not show the same anticancer effect in all patients.

Meanwhile, the intestine of the human body is a symbiotic place for various types of bacteria, and has an important function of absorbing nutrients according to the digestion of food and excreting unnecessary materials from the body. Further, changes in the environment caused by intestinal bacteria are directly linked to intestinal immunity, which ultimately affects the body's immunity. As research on microorganisms beneficial to such intestinal health has been continuously conducted for many years, research results on the involvement of intestinal microbes in immunity and metabolic activity *in vivo* have been actively published worldwide. Probiotics are live microorganisms that improve the *in vivo* immune response by improving the imbalance in the digestive system, suppressing harmful bacteria, and enhancing natural defense effects. To date, functional studies on probiotics for improving immune function have been conducted according to the taxonomic morphology of species and genus using *Lactobacillus, Lactococcus, Bifidobacterium,* and the like.

However, it has never been shown that a *Prevotella stercorea* strain enhances the efficacy of immune anticancer agents through immune enhancing effects.

### [Disclosure]

### [Technical Problem]

A technical problem to be solved by the present invention is to provide a composition for enhancing immunity and a composition for increasing anticancer efficacy, including a *Prevotella stercorea* strain or a culture solution thereof as an active ingredient, and the present inventors confirmed that the *Prevotella stercorea* strain increases the proliferation of T cells, increases the IFN-γ secretion of T cells, and increases the anticancer efficacy of immune barrier inhibitors.

Thus, an object of the present invention is to provide a composition for enhancing immunity, comprising a *Prevotella stercorea* strain or a culture solution thereof as an active ingredient.

Another object of the present invention is to provide a pharmaceutical composition for increasing an anticancer effect, comprising a *Prevotella stercorea* strain or a culture solution thereof as an active ingredient.

Still another object of the present invention is to provide a pharmaceutical composition for preventing or treating cancer, comprising a *Prevotella stercorea* strain or a culture solution thereof; and an immune checkpoint inhibitor as an active ingredients.

However, the technical problems to be achieved by the present invention are not limited to the aforementioned problems, and other problems that are not mentioned may be clearly understood by those skilled in the art from the following description.

### [Technical Solution]

To solve the above problems, the present invention provides a composition for enhancing immunity, comprising a *Prevotella stercorea* strain or a culture solution thereof as an active ingredient.

In an exemplary embodiment of the present invention, the *Prevotella stercorea* strain may be deposited under the accession number KCTC15079, but is not limited thereto.

In addition, the present invention provides a pharmaceutical composition for increasing an anticancer effect, comprising a *Prevotella stercorea* strain or a culture solution thereof as an active ingredient.

Furthermore, the present invention provides a food composition for increasing an anticancer effect, comprising a *Prevotella stercorea* strain or a culture solution thereof as an active ingredient.

Further, the present invention provides a pharmaceutical composition for preventing or treating cancer, comprising a *Prevotella stercorea* strain or a culture solution thereof; and an immune checkpoint inhibitor as an active ingredient.

In an exemplary embodiment of the present invention, the immune checkpoint inhibitor may be a PD-L1 inhibitor or a PD-1 inhibitor, but is not limited thereto.

In another exemplary embodiment of the present invention, the PD-L1 inhibitor may be selected from the group consisting of atezolizumab, avelumab, durvalumab, envafolimab, cosibelimab, AUNP12, CA-170 and BMS-986189, but is not limited thereto.

In still another exemplary embodiment of the present invention, the PD-1 inhibitor may be selected from the group consisting of nivolumab, pembrolizumab, cemiplimab, spartalizumab, camrelizumab, sintilimab, tislelizumab, toripalimab, dostarlimab, INCMGA00012, AMP-224 and AMP-514, but is not limited thereto.

In yet another exemplary embodiment of the present invention, the *Prevotella stercorea* strain or a culture solution thereof; and an immune checkpoint inhibitor may be simultaneously administered, but are not limited thereto.

In yet another exemplary embodiment of the present invention, the *Prevotella stercorea* strain or a culture solution thereof; and an immune checkpoint inhibitor may be sequentially administered, but are not limited thereto.

In yet another exemplary embodiment of the present invention, the *Prevotella stercorea* strain or a culture solution thereof may increase the proliferation of CD4⁺ T cells and CD8⁺ T cells, but is not limited thereto.

In yet another exemplary embodiment of the present invention, the *Prevotella stercorea* strain or a culture solution thereof may increase the interferon-γ (IFN-γ) secretion of T cells, but is not limited thereto.

In yet another exemplary embodiment of the present invention, the cancer may be one or more selected from the group consisting of breast cancer, colorectal cancer, lung cancer, small cell lung cancer, gastric cancer, liver cancer, hematologic malignancy, bone cancer, pancreatic cancer, skin cancer, head or neck cancer, cutaneous or intraocular melanoma, uterine cancer, ovarian cancer, rectal cancer, cancer near the anus, colon cancer, fallopian tube carcinoma, endometrial carcinoma, cervical cancer, vaginal cancer, vulva carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphotic lymphoma, bladder cancer, renal cancer, ureter cancer, renal cell carcinoma, renal pelvis carcinoma, CNS tumors, primary CNS lymphoma, spinal cord tumors, brainstem glioma and pituitary adenoma, but is not limited thereto.

Further, the present invention provides a method for enhancing immunity, the method comprising administering a composition comprising a *Prevotella stercorea* strain or a culture solution thereof to a subject in need thereof.

In addition, the present invention provides a use of a composition comprising a *Prevotella stercorea* strain or a culture solution thereof for enhancing immunity.

Furthermore, the present invention provides a use of a *Prevotella stercorea* strain or a culture solution thereof for producing an immune enhancer.

Further, the present invention provides a method for increasing an anticancer effect, the method comprising administering a composition comprising a *Prevotella stercorea* strain or a culture solution thereof to a subject in need thereof.

In addition, the present invention provides a use of a composition comprising a *Prevotella stercorea* strain or a culture solution thereof for increasing an anticancer effect.

Furthermore, the present invention provides a use of a *Prevotella stercorea* strain or a culture solution thereof for producing an agent for increasing an anticancer effect.

Further, the present invention provides a method for preventing or treating cancer, the method comprising administering a pharmaceutical composition comprising a *Prevotella stercorea* strain or a culture solution thereof; and an immune checkpoint inhibitor as an active ingredients to a subject in need thereof.

In addition, the present invention provides a use of a pharmaceutical composition comprising a *Prevotella stercorea* strain or a culture solution thereof; and an immune checkpoint inhibitor as an active ingredients for preventing or treating cancer.

Furthermore, the present invention provides a use of a *Prevotella stercorea* strain or a culture solution thereof; and an immune checkpoint inhibitor for producing a therapeutic agent for cancer.

### [Advantageous Effects]

The present inventors confirmed that a *Prevotella stercorea* strain increases the proliferation of T cells and increases the IFN-γ secretion of T cells, and *Prevotella stercorea* increases the anticancer efficacy of immune checkpoint inhibitors. Therefore, it is expected that a *Prevotella stercorea* strain or a culture solution thereof can be usefully used for enhancing immunity and increasing the anticancer effect of immune checkpoint inhibitors.

### [Description of Drawings]

FIG. 1 illustrates the proliferation effect of T cells when the cells are treated with a *Prevotella stercorea* strain culture supernatant.
FIG. 2 illustrates the proliferation effect of CD4⁺ T cells when the cells are treated with a *Prevotella stercorea* strain culture supernatant.
FIG. 3 illustrates the proliferation effect of CD8⁺ T cells when the cells are treated with a *Prevotella stercorea* strain culture supernatant.
FIG. 4 illustrates IFN-γ secreted from CD4⁺ T cells and CD8⁺ T cells, which are treated with a *Prevotella stercorea* strain culture supernatant.
FIG. 5 illustrates IFN-γ secreted from CD8⁺ T cells treated with a *Prevotella stercorea* strain culture supernatant.
FIG. 6 shows that the combined use of a *Prevotella stercorea* strain and α-PD1 antibody reduces the growth of colon cancer tumors.
FIG. 7 shows that the combined use of a *Prevotella stercorea* strain and α-PD1 antibody increases the memory T cell proportion of CD8+ T cells in the spleen and tumors.

### [Best Modes of the Invention]

In an exemplary embodiment of the present invention, it was confirmed that during treatment with a *Prevotella stercorea* strain culture supernatant, the degree of proliferation of T cells was significantly increased in a concentration-dependent manner to the stain culture solution, and the degree of proliferation of CD4⁺ or CD8⁺ T cells was significantly increased.

In another exemplary embodiment of the present invention, it was confirmed that during treatment with a *Prevotella stercorea* strain culture supernatant, the IFN-γ secretion of CD4⁺ T cells and CD8⁺ T cells was significantly increased.

In still another exemplary embodiment of the present invention, it was confirmed that when a *Prevotella stercorea* strain and a PD-1 inhibitor were co-administered, the effect of suppressing the growth of tumors was remarkably excellent, and the memory T cell proportion of CD8⁺ T cells in intestinal cells and tumor-infiltrating lymphocytes (TILs) was significantly increased by the combined use of a *Prevotella stercorea* strain culture solution and a PD-1 inhibitor.

Therefore, the *Prevotella stercorea* strain of the present invention or a culture solution thereof may be usefully used for enhancing immunity and increasing an anticancer effect, and is provided as an anticancer composition used in combination with an immune checkpoint inhibitor based on synergistic effects with the immune checkpoint inhibitor.

Hereinafter, the present invention will be described in detail.

The present invention relates to a composition for enhancing immunity and a pharmaceutical composition for increasing an anticancer effect, comprising a *Prevotella stercorea* strain or a culture solution thereof as an active ingredient.

Further, the present invention relates to a pharmaceutical composition for preventing or treating cancer, comprising a *Prevotella stercorea* strain or a culture solution thereof; and an immune checkpoint inhibitor as an active ingredients.

In the present invention, the *Prevotella stercorea* strain may be deposited under the accession number KCTC15079, but is not limited thereto.

As used herein, the term 'culture' refers to all actions that are performed for growing microorganisms under appropriately artificially controlled environmental conditions, and is a concept including 'fermentation' in the present invention.

In the present invention, the bacterial cells of the '*Prevotella stercorea*' include not only live cells obtained from the culture medium, but also any processed forms of lactic acid bacteria known to those skilled in the art, and include, for example, bacterial lysate products, dried products, frozen products, and the like, but are not limited thereto.

In the present invention, the meaning of the term `culture solution' includes `fermentation liquid,' and includes a culture solution itself cultured in a liquid medium, and processed products derived from a culture solution such as a filtrate (centrifuged supernatant) in which a strain is removed by filtering or centrifuging the above culture solution, but is not limited thereto.

In the present invention, the immune checkpoint inhibitor may be a PD-L1 inhibitor or a PD-1 inhibitor, but is not limited thereto.

In the present invention, the PD-L1 inhibitor may be an anti-PD-L1 antibody, but is not limited thereto.

In the present invention, the PD-1 inhibitor may be an anti-PD-1 antibody, but is not limited thereto.

In the present invention, the PD-L1 inhibitor may be selected from the group consisting of atezolizumab, avelumab, durvalumab, envafolimab, cosibelimab, AUNP12, CA-170 and BMS-986189, but is not limited thereto.

In the present invention, the PD-1 inhibitor may be selected from the group consisting of nivolumab, pembrolizumab, cemiplimab, spartalizumab, camrelizumab, sintilimab, tislelizumab, toripalimab, dostarlimab, INCMGA00012, AMP-224 and AMP-514, but is not limited thereto.

In the present invention, the *Prevotella stercorea* strain or a culture solution thereof; and an immune checkpoint inhibitor may be mixed at a volume ratio of 0.5 to 10:1, 0.5 to 9:1, 0.5 to 8:1, 0.5 to 7:1, 0.5 to 6:1, 0.5 to 10:1, 0.5 to 5:1, 0.5 to 4.5:1, 1 to 10:1, 1 to 9:1, 1 to 8:1, 1 to 7:1, 1 to 6:1, 1 to 5:1, 1 to 4.5:1, 2 to 10:1, 2 to 9:1, 2 to 8:1, 2 to 7:1, 2 to 6:1, 2 to 5:1, 2 to 4.5:1, 3 to 10:1, 3 to 9:1, 3 to 8:1, 3 to 7:1, 3 to 6:1, 3 to 5:1, 3 to 4.5:1, 3.5 to 4.5:1 or 4:1, but are not limited thereto. In addition, the *Prevotella stercorea* strain or a culture solution thereof; and an immune checkpoint inhibitor may be individually administered at the above volume ratio, but are not limited thereto.

In the present invention, the *Prevotella stercorea* strain or a culture solution thereof may be administered simultaneously or sequentially with the immune checkpoint inhibitor, but are not limited thereto. In the present invention, the composition including the *Prevotella stercorea* strain or a culture solution thereof as an active ingredient may be formulated to be present in one container in the form of a mixture with an immune checkpoint inhibitor, and may be formulated such that they are present in separate containers and administered simultaneously or sequentially. Furthermore, the composition including the *Prevotella stercorea* strain or a culture solution thereof as an active ingredient may be administered before the treatment of the immune checkpoint inhibitor, and may be administered as a secondary therapy after the treatment of the immune checkpoint inhibitor, but is not limited thereto.

As used herein, the term "cancer" refers to a disease related to the regulation of cell death, and refers to a disease caused by hyperproliferation of cells when the balance for normal cell apoptosis is broken. In some cases, such abnormal hyperproliferative cells may invade surrounding tissues and organs to form a mass and cause destruction or deformation of the normal structures in the body, and these conditions are collectively referred to as tumors. In general, tumors may be classified into benign tumors and malignant tumors. A malignant tumor has a very rapid growth rate relative to a benign tumor, and causes metastasis when invading surrounding tissues, thus becoming life-threatening. Such malignant tumor is typically referred to as cancer.

In the present invention, the cancer may be one or more selected from the group consisting of breast cancer, colorectal cancer, lung cancer, small cell lung cancer, gastric cancer, liver cancer, hematologic malignancy, bone cancer, pancreatic cancer, skin cancer, head or neck cancer, cutaneous or intraocular melanoma, uterine cancer, ovarian cancer, rectal cancer, cancer near the anus, colon cancer, fallopian tube carcinoma, endometrial carcinoma, cervical cancer, vaginal cancer, vulva carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphotic lymphoma, bladder cancer, renal cancer, ureter cancer, renal cell carcinoma, renal pelvis carcinoma, CNS tumors, primary CNS lymphoma, spinal cord tumors, brainstem glioma and pituitary adenoma, but is not limited thereto.

In the present invention, "immune enhancement" means increasing the immune response or activity of the *in vivo* immune system. Immunity is broadly divided into innate immunity and acquired immunity. Innate immunity does not have a special memory effect for antigens, and thus functions to protect against the invasion of antigens by reacting non-specifically without a special memory action. Many anatomical defensive actions and biological actions such as complements present in skin, mucous tissue, strongly acidic gastric acid, and blood participate in innate immunity, and examples of cells responsible for innate immunity include macrophages and polymorphonuclear leukocytes that act as phagocytes, natural killing cells (NK cells) that kill infected cells and cancer cells, and the like. Acquired immunity is a stronger immune response including immunological memory, which is the function of remembering antigen labels, and needs the recognition of non-self antigens through an antigen-specific antigen presentation process.

In the present invention, "the increase of an anticancer effect" may increase the tumor growth inhibitory effect of an anticancer agent when used in combination with the anticancer agent, but is not limited thereto. In an exemplary embodiment of the present invention, as a result of co-administering an anti-PD-1 antibody, which is an immune checkpoint inhibitor, and a *Prevotella stercorea* strain culture supernatant, it was confirmed that not only was the growth of colon cancer suppressed, but also the proportion of memory T cells was significantly increased (see Example 3 of the present invention).

Since the *Prevotella stercorea* strain of the present invention promotes the proliferation of T cells and the effect of promoting IFN-γ secretion of T cells is far superior to other strains, the *Prevotella stercorea* strain has an effect of increasing innate immunity and required immune functions.

The content of the *Prevotella stercorea* strain or a culture solution thereof in the composition of the present invention can be appropriately adjusted according to the symptoms of the disease, the degree of progression of the symptoms, the condition of the patient, and the like, and may be, for example, 0.0001 to 99.9 wt%, or 0.001 to 50 wt%, but is not limited thereto. The content ratio is a value based on a dry amount from which the solvent is removed.

The pharmaceutical composition of the present invention may further include an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, an external preparation such as a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be included in the composition according to the present invention include lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, or Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, or polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, sucrose, magnesium aluminum silicate, a D-sorbitol solution, or light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, or light anhydrous silicic acid.

As an additive for liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ethers, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In a syrup according to the present invention, a white sugar solution, other sugars or sweeteners, and the like may be used, and a fragrance, a colorant, a preservative, a stabilizer, a suspending agent, an emulsifier, a thickener, and the like may be used if necessary.

Purified water may be used for the emulsion according to the present invention, and an emulsifier, a preservative, a stabilizer, a fragrance, and the like may be used if necessary.

In the suspension according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose, HPMC 1828, HPMC 2906, and HPMC 2910 may be used, and a surfactant, a preservative, a stabilizer, a colorant, and a fragrance may be used if necessary.

The injection according to the present invention may include: a solvent such as distilled water for injection, a 0.9% sodium chloride injection, a Ringer's injection, a dextrose injection, a dextrose+sodium chloride injection, PEG, a lactated Ringer's injection, ethanol, propylene glycol, non-volatile oil-sesame oil, cottonseed oil, peanut oil, corn oil, ethyl oleate, isopropyl myristate, or benzoic acid benzene; a solubilizing aid such as sodium benzoate, sodium salicylate, sodium acetate, urea, urethane, monoethyl acetamide, butazolidin, propylene glycol, Tweens, nijungtinateamide, hexamine, or dimethylacetamide; a buffer such as a weak acid or salt thereof (acetic acid or sodium acetate), a weak base or salt thereof (ammonia or ammonium acetate), an organic compound, a protein, albumin, peptone, or a gum; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), or ethylenediaminetetraacetic acid; a sulfating agent such as 0.1% sodium bisulfide, sodium formaldehydesulfoxylate, thiourea, disodium ethylenediaminetetraacetate, or acetone sodium bisulfite; an analgesic such as benzyl alcohol, chlorobutanol, procaine hydrochloride, glucose, or calcium gluconate; and a suspending agent such as carboxymethyl cellulose sodium, sodium alginate, Tween 80, or aluminum monostearate.

In a suppository according to the present invention, it is possible to use a base such as cacao butter, lanolin, Witepsol, polyethylene glycol, glycerogelatin, methylcellulose, carboxymethyl cellulose, a mixture of stearic acid and oleic acid, Subanal, cottonseed oil, peanut oil, palm oil, cacao butter+cholesterol, lecithin, ranetwax, glycerol monostearate, Tween or Span, Imhausen, monolen (propylene glycol monostearate), glycerin, Adeps solidus, Buytyrum Tego-G, Cebes Pharma 16, hexalide base 95, Cotomar, Hydroxote SP, S-70-XXA, S-70-XX75(S-70-XX95), Hydrokote 25, Hydrokote 711, idropostal, Massa estrarium (A, AS, B, C, D, E, I, T), Massa-MF, Masupol, Masupol-15, Neosupostal-ene, Paramound-B, Suposhiro (OSI, OSIX, A, B, C, D, H, L), suppository base IV types (AB, B, A, BC, BBG, E, BGF, C, D, 299), Supostal (N, Es), Wecobee (W, R, S, M ,Fs), or a tegester triglyceride base (TG-95, MA, 57).

A solid formulation for oral administration includes a tablet, a pill, a powder, a granule, a capsule, or the like, and the solid formulation is prepared by mixing at least one excipient, for example, starch, calcium carbonate, sucrose or lactose, gelatin, and the like, with an extract. Further, in addition to a simple excipient, lubricants such as magnesium stearate and talc are also used.

A liquid formulation for oral administration corresponds to a suspension, a liquid for internal use, an emulsion, a syrup, or the like, and the liquid formulation may include, in addition to water and liquid paraffin which are simple commonly used diluents, various excipients, for example, a wetting agent, a sweetener, a fragrance, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including the type of disease of a patient, the severity of the disease, the activity of drugs, sensitivity to drugs, administration time, administration route, excretion rate, treatment period, and simultaneously used drugs, and other factors well known in the medical field.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

The pharmaceutical composition of the present invention may be administered to a subject in need via various routes. All methods of administration may be expected, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spray via the mouth or nose, skin administration, transdermal administration, and the like.

The pharmaceutical composition of the present invention is determined by the type of drug that is an active ingredient, as well as various related factors such as the disease to be treated, the route of administration, the age, sex, and body weight of a patient, and the severity of the disease.

As used herein, the individual is a subject in need of treatment of a disease, and more specifically, may be a mammal such as a human or a non-human primate, a mouse, a rat, a dog, a cat, a horse, or a cow, but is not limited thereto.

"Administration" as used herein refers to the provision of a predetermined composition of the present invention to a subject in need thereof by any suitable method.

As used herein, "prevention" refers to all actions that suppress or delay the onset of a target disease, and "treatment" refers to all actions that ameliorate or beneficially change a target disease and the resulting metabolic abnormalities by administration of the pharmaceutical composition according to the present invention, and "amelioration" refers to all actions that reduce a target disease and associated parameters, for example, the severity of symptoms, by administration of the composition according to the present invention.

Furthermore, the present invention provides a food composition for enhancing immunity or increasing an anticancer effect, comprising a *Prevotella stercorea* strain or a culture solution thereof as an active ingredient.

When the *Prevotella stercorea* strain of the present invention or a culture solution thereof is used as a food additive, the *Prevotella stercorea* strain or a culture solution thereof may be added as it is or used with another food or other food ingredients, and may be appropriately used according to a typical method. The amount of active ingredient mixed may be suitably determined according to the purpose of use (prevention, health or therapeutic treatment). In general, when a food or beverage is prepared, the *Prevotella stercorea* strain of the present invention or a culture solution thereof may be added in an amount of 15 wt% or less, or 10 wt% or less based on the raw material. However, in the case of long-term intake for the purpose of health and hygiene or for the purpose of controlling health, the amount may be less than the above range, and the effective ingredient may be used in an amount above this above range because there is no problem in terms of safety.

The type of food is not particularly limited. Examples of food to which the material may be added include meat, sausage, bread, chocolate, candy, snacks, confectioneries, pizza, instant noodles, other noodles, gum, dairy products including ice cream, various soups, beverages, tea, drinks, alcoholic beverages, vitamin complexes, and the like, and include all health functional foods in a typical sense.

The health beverage composition according to the present invention may contain various flavors or natural carbohydrates and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin or stevia extract, a synthetic sweetener such as saccharin or aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not very important, but is generally selected within a range of 0.01 to 0.20 parts by weight per 100 parts by weight of the composition of the present invention.

Since the present invention may be modified into various forms and include various exemplary embodiments, specific exemplary embodiments will be illustrated in the drawings and described in detail in the Detailed Description. However, the description is not intended to limit the present invention to the specific exemplary embodiments, and it is to be understood that all the changes, equivalents, and substitutions belonging to the spirit and technical scope of the present invention are included in the present invention. When it is determined that the detailed description of the related publicly known art in describing the present invention may obscure the gist of the present invention, the detailed description thereof will be omitted.

### [Modes of the Invention]

Hereinafter, preferred examples for helping with understanding of the present invention will be suggested. However, the following examples are provided only so that the present invention may be more easily understood, and the content of the present invention is not limited by the following examples.

### [Examples]

### Experimental Example 1. Experimental method

### Experimental Example 1-1. Preparation of mice

All animal experiments were performed according to protocols approved by the Animal Care and Use Committee of the Gwangju Institute of Science and Technology (GIST). All animals used in this study were maintained and handled according to the approved policies of the study protocol. The number of mice assigned to strain administration was sufficient to provide statistical results including colon cancer tumor size, cytokines and immune cell profiling. All *in vivo* studies were not blinded, and mice were randomly selected for each group prior to administration of the strain of *Prevotella stercorea* (*P. stercorea*, KTCT 15079). Wild-type C57B6/N (female) mice were provided by Orient Bio.

### Experimental Example 1-2. Preparation of Prevotella stercorea strain culture supernatant

*Prevotella stercorea* KTCT 15079 was cultured in a medium made of the following composition. A liquid medium included a brain heart leaching medium (37 g/1 L), 0.05% L-cysteine HCl (0.5 g/1 L), a 0.5% yeast extract (5 g/1 L), 1% hemin stock (10 ml/1 L), and 0.02% vitamin K1 (0.2 ml/1 L). The hemin stock (Sigma, 51280) was used at a concentration of 0.5 mg/mL, and the vitamin K1 stock (Sigma, 95271) was used at a concentration of 0.1 mL/20 mL. The cultured strain was used when OD600 = 2 to 4.

The culture supernatant used for the *in vitro* cell experiment was used by inoculating 1 mL of the strain culture solution into 10 mL of RPMI medium and culturing for one day when the cultured strain was OD600 = 2 to 4, and then isolating the supernatant. The corresponding culture supernatant was treated with 1, 5, or 10% (v/v) of a cultured solution of immune cells isolated from human peripheral blood mononuclear cells (PBMCs), and cultured for 3 days.

In order to obtain the strain used in the *in vivo* animal experiment, when the cultured strain was OD600 = 2 to 4, it was centrifuged at 6000 rpm for 5 minutes to isolate the strain in the form of a pellet. The isolated strain was diluted with PBS and used to set OD600 = 4. 200 mL of the corresponding strain was orally administered to each mouse at a cycle of 6 days/week.

### Experimental Example 1-3. Colon cancer tumor model mouse preparation and tumor growth measurement

In the case of a syngeneic tumor mouse model, the *Prevotella stercorea* strain was diluted with PBS 2 weeks before tumor inoculation in female C57B6/N mice (wild-type, 6-week-old) to set OD600 = 4, 200 mL of the diluted strain was orally administered at a cycle of 6 days/weeks, and administration was continued throughout the duration of the experiment. The tumor mouse model was constructed by subcutaneously injecting 2 × 10⁵ MC38 colon cancer cells into female C57B6/N mice. The tumor size was measured 3 times a week until the end of the experiment, and the tumor volume was calculated as length × width² × 0.5 (unit: mm³). After inoculation of tumor cells, an anti-PD-1 monoclonal antibody (mAb; clone RMP1-14, BioXCell) in PBS was intraperitoneally injected into the tumor mouse model at 2 mg/kg on days 3, 7, 10, 14, 17 and 21.

### Experimental Example 1-4. Human T cell analysis

The response of human T cells to the strain was analyzed. Blood samples were collected from healthy donors with informed consent at the GIST in Korea. This study was approved by the Institutional Review Board (20192009-BR-48-02-04). Peripheral blood mononuclear cells (PBMCs) were purified using Ficoll-Paque Plus (GE Healthcare Life Sciences) density gradient centrifugation. For the experiment, *P. stercorea*, *Bacteroides plebeius,* and *Ligilactobacillus salivarius* were purchased from KCTC. Each strain was cultured according to the manufacturer's instructions. Autologous CD4⁺ or CD8⁺ T cells were isolated from the remaining PBMCs using a negative immunomagnetic selection kit, and 5×10⁴ cells were suspended in a well including RPMI 1640, 10% human AB⁺ serum, 1% 2 mM glutamine, 20 IU of IL-2 per ml, 1,000 IU granulocyte-macrophage colony stimulating factor (Miltenyi Biotec), 1% penicillin-streptomycin (Gibco/Invitrogen), and 50 µg/ml gentamicin (Gibco/Invitrogen). Then, autologous CD4⁺ or CD8⁺ T cells were stained with carboxyfluorescein diacetate succinimidyl ester (CSFE) (Thermo). Stained autologous CD4⁺ or CD8⁺ T cells were distributed in 96-well plates at 5×10⁴ cells per well and then treated either alone or with a supernatant of *P. stercorea*, *B. plebeius* or *L. salivarius,* and cultured at a final concentration of 10% under the conditions of 5% CO₂ and 37°C for 2 hours.

Autologous CD4⁺ or CD8⁺ T cells were incubated under the conditions of 5% CO₂ and 37°C for 48 hours. After 48 hours, the supernatant was collected and stored at -20°C, and IFN-γ levels were measured using a Human IFN-γ ELISA kit (invitrogen). Cultured autologous CD4⁺ or CD8⁺ T cells were collected and washed twice using phosphate buffered saline (PBS) (Thermo). The proliferation of CD4⁺ or CD8⁺ T cells was measured by flow cytometry.

### Experimental Example 1-5. Flow cytometry

To confirm the proliferation effect of T cells, cells stained with CANTO II (BD Bioscience) were obtained using BDFACS Divasoftware v.8.0.2 (BD Bioscience). T cells stained with CFSE were gated with side scatter (SSC) and forward scatter (FSC) values. Then, isolated cell regions were gated with FSC-H and FSC-A values to find single cell populations. Proliferated cell populations were observed by measuring single cells with FITC. Data analysis was performed using FlowJo software (v.10, TreeStar).

In order to isolate immune cells within tumors, the tumors were first dissociated. Dissected tumors were cut into small pieces, and transferred to RPMI 1640 medium (Gibco/Invitrogen) supplemented with 2.5 mg/ml collagenase type 1, 1.5 mg/ml collagenase type 2, 1 mg/ml collagenase type 4, 50 µg/ml DNase type 1, and 0.25 mg/ml hyaluronidase type IV-S. Samples were incubated at 37°C for 50 minutes and then filtered through a 70 µm cell strainer (BD Bioscience).

Spleens were crushed in RPMI 1640 medium, cultured in an RBC lysis buffer (eBioscience), and filtered through a 70 µm cell strainer. To block Fc receptors, spleen and tumor cells were incubated with anti-mouse CD16/CD32 (BD Bioscience) at 4°C for 10 minutes. After viability check and surface staining, a fixed/permeable buffer set (BioLegend) solution was added. The following anti-mouse antibodies were used for spleen and tumor immune cell profiling: CD45 (1:100 dilution; BioLegend, 103116); CD3 (1:20 dilution; BioLegend, 100218); NK1.1 (1:100 dilution; BioLegend, 108708); CD49b (1:100 dilution; BioLegend, 108910); CD4 (1:100 dilution; BioLegend, 100422); CD25 (1:20 dilution; BioLegend, 101904); FOXP3 (1:20 dilution; Invitrogen, 17-5773-82); CD44 (1:100 dilution; BioLegend, 103008); CD62L (1:100 dilution; BioLegend, 104412); CD8a (1:25 dilution; BioLegend, 100706, spleen and tumor); CD8a (1:80 dilution; BioLegend, 100712, intestine cytokine expression); IFN-γ (1:50 dilution; BioLegend, 505806); and IL-2 (1:200 dilution; BioLegend, 503820). Stained cells were obtained with CANTO II (BD Bioscience) using BD FACSDiva software v.8.0.2 (BD Bioscience), and data analysis was performed using FlowJo software (v10, Tree Star).

### Experimental Example 1-6. Memory T cell analysis method

CD8⁺ T cells were gated by markers with CD3⁺, CD45⁺ and CD8⁺. Memory T cells of CD8⁺ T cells were gated by additional markers with CD44⁺ and CD62L⁺. Therefore, the ratio of memory T cells/CD8⁺ T cells was confirmed as the ratio of the number of CD3⁺, CD45⁺, CD8⁺, CD44⁺ and CD62L⁺ cells/the number of CD3⁺, CD45⁺ and CD8⁺ cells.

### Example 1. Confirmation of effect of increasing proliferation of T cells according to treatment with Prevotella stercorea strain culture supernatant

CD4⁺ or CD8⁺ T cells, which remember cancer cells as antigens, play an important role in maintaining immunity to cancer cells. Accordingly, it was confirmed whether the *Prevotella stercorea* strain affects the proliferation of T cells.

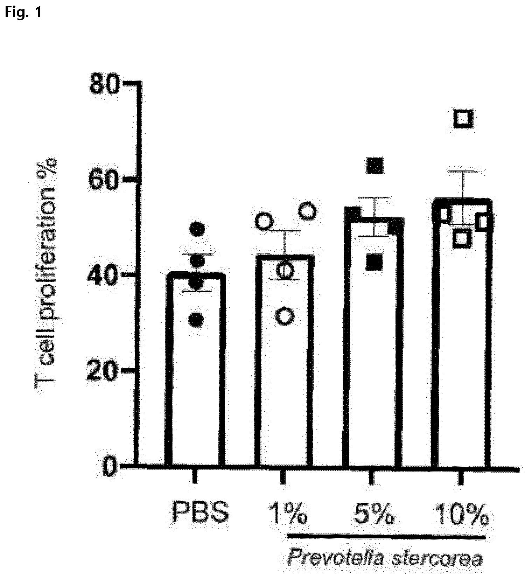

As illustrated in FIG. 1, it was confirmed that when the cells were treated with a *Prevotella stercorea* strain culture supernatant, the degree of proliferation of T cells was significantly increased in a concentration-dependent manner of the strain culture supernatant.

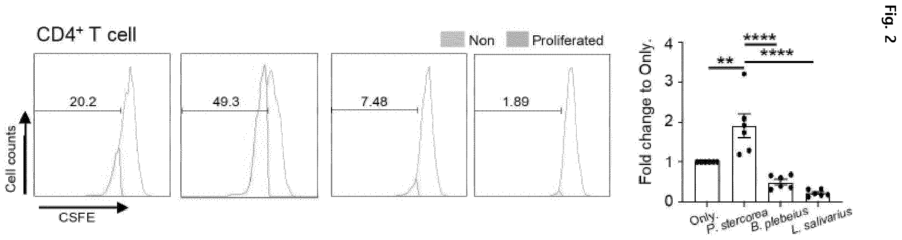

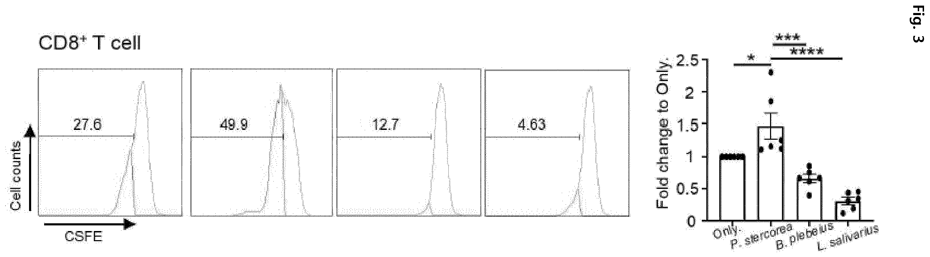

More specifically, as illustrated in FIGS. 2 and 3, it was confirmed that when the cells were treated the *Prevotella stercorea* strain culture supernatant at a concentration of 10% (v/v), CD4 + or CD8 + T cells, the degree of proliferation of CD4⁺ or CD8⁺ T cells was significantly increased compared to treatment with other strains.

### Example 2. Confirmation of effect of increasing IFN-γ secretion of T cells according to treatment with Prevotella stercorea strain culture supernatant

T cells including CD4 help the antibody response and the role of CD8-expressing T cells, and secrete IFN-γ to create an inflammatory environment to facilitate anticancer immunity. Accordingly, it was confirmed whether CD4⁺ or CD8⁺ T cells induced the secretion of IFN-γ by treatment with the *Prevotella stercorea* strain. Blood-derived PBMCs were isolated from a total of 6 donors from D1 to D6, and an experiment was performed by isolating CD8⁺ T cells from the PBMCs of the corresponding patients.

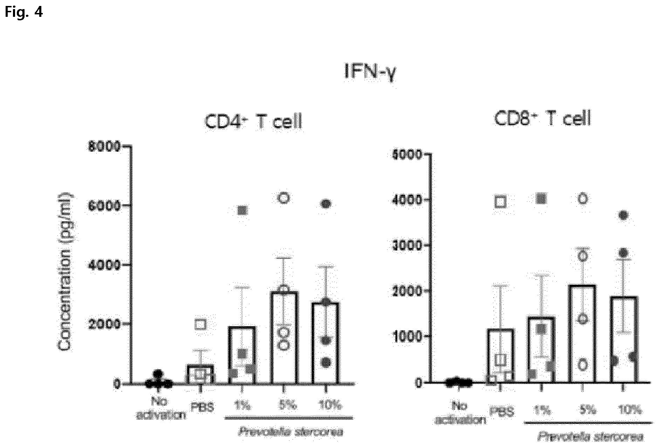

As illustrated in FIG. 4, it was confirmed that during treatment with the *Prevotella stercorea* strain culture supernatant, the IFN-γ secretion of CD4⁺ T cells and CD8⁺ T cells was significantly increased.

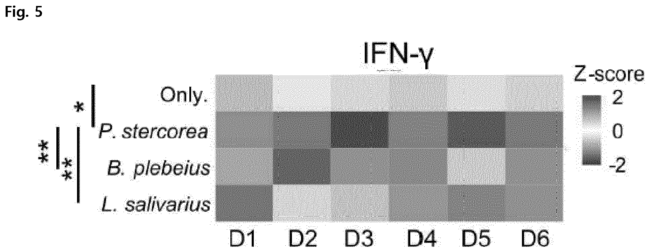

In addition, as illustrated in FIG. 5, the higher the Z-score, the higher the concentration of IFN-γ secreted by CD8⁺ T cells of the same test subject (donor), so it was confirmed that the IFN-γ secretion of CD8⁺ T cells treated with 10% (v/v) concentration of the *Prevotella stercorea* strain culture supernatant was remarkably increased compared to the control. Meanwhile, in the case of the culture supernatant of the *Bacteroides plebeius* and *Ligilactobacillus salivarius* strains, it was confirmed that IFN-γ secretion actually decreased.

### Example 3. Confirmation of colon cancer growth inhibitory effect according to combined use of Prevotella stercorea strain and PD-1 inhibitor

Effect on cytokine secretion In the body, the spleen is an organ that is responsible for the main protective immune response against antigens derived from blood, and is a major lymphoid organ where B and T lymphocytes mature and lymphocytes are differentiated by antigen stimulation. Therefore, the proliferation of splenocytes has a very important meaning in the immune system. In particular, splenocytes are closely related to immune response, and the size or number thereof may be used as a direct index, and therefore it is used as a representative immune index. In addition, it is known that the anticancer response is enhanced due to the role of memory T cells, and cancer-infiltrating lymphocytes expressing CD4 or CD8 are known to exhibit the characteristics of memory T cells. In the method described in Experimental Example 1-3, the *Prevotella stercorea* strain and the PD-1 inhibitor were co-administered.

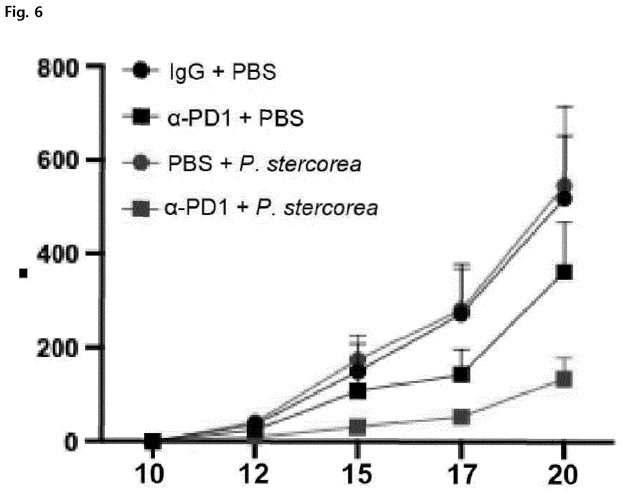

As illustrated in FIG. 6, it was confirmed that when the *Prevotella stercorea* strain and the PD-1 inhibitor were co-administered, the tumor volume growth inhibitory effect was remarkably excellent. In contrast, the effects of the negative IgG control, the PD-1 inhibitor single administration group, and the *Prevotella stercorea* strain single administration group were insignificant.

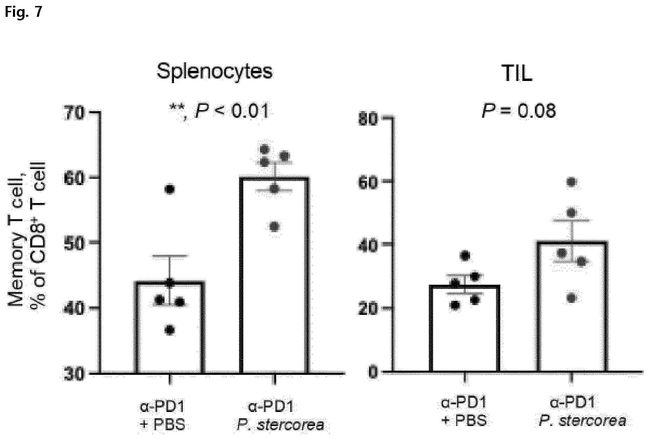

Furthermore, as illustrated in FIG. 7, it was confirmed that the memory T cell proportion of CD8⁺ T cells in splenocytes and tumor infiltrating lymphocytes (TILs) was significantly increased according to the combined use of the *Prevotella stercorea* strain and the PD-1 inhibitor.

The above description of the present invention is provided for illustrative purposes, and those skilled in the art to which the present invention pertains will understand that the present invention can be easily modified into other specific forms without changing the technical spirit or essential features of the present invention. Therefore, it should be understood that the above-described examples are only illustrative in all aspects and not restrictive.

### [Industrial Applicability]

The present inventors confirmed that a *Prevotella stercorea* strain increases the proliferation of T cells and increases the IFN-γ secretion of T cells, and that *Prevotella stercorea* increases the anticancer efficacy of immune checkpoint inhibitors. Therefore, a *Prevotella stercorea* strain or a culture solution thereof can be usefully used for enhancing immunity and increasing the anticancer effect of immune checkpoint inhibitors, and thus has industrial applicability.

## Claims

1. A composition for enhancing immunity, comprising a *Prevotella stercorea* strain or a culture solution thereof as an active ingredient.

2. The composition of claim 1, wherein the *Prevotella stercorea* strain is deposited under the accession number KCTC15079.

3. A pharmaceutical composition for increasing an anticancer effect, comprising a *Prevotella stercorea* strain or a culture solution thereof as an active ingredient.

4. The pharmaceutical composition of claim 3, wherein the *Prevotella stercorea* strain is deposited under the accession number KCTC15079.

5. A food composition for increasing an anticancer effect, comprising a *Prevotella stercorea* strain or a culture solution thereof as an active ingredient.

6. A pharmaceutical composition for preventing or treating cancer, comprising a *Prevotella stercorea* strain or a culture solution thereof; and an immune checkpoint inhibitor as an active ingredients.

7. The pharmaceutical composition of claim 6, wherein the immune checkpoint inhibitor is a PD-L1 inhibitor or a PD-1 inhibitor.

8. The pharmaceutical composition of claim 7, wherein the PD-L1 inhibitor is selected from the group consisting of atezolizumab, avelumab, durvalumab, envafolimab, cosibelimab, AUNP12, CA-170 and BMS-986189.

9. The pharmaceutical composition of claim 7, wherein the PD-1 inhibitor is selected from the group consisting of nivolumab, pembrolizumab, cemiplimab, spartalizumab, camrelizumab, sintilimab, tislelizumab, toripalimab, dostarlimab, INCMGA00012, AMP-224 and AMP-514.

10. The pharmaceutical composition of claim 6, wherein the *Prevotella stercorea* strain or a culture solution thereof; and the immune checkpoint inhibitor are simultaneously administered.

11. The pharmaceutical composition of claim 6, wherein the *Prevotella stercorea* strain or a culture solution thereof; and the immune checkpoint inhibitor are sequentially administered.

12. The pharmaceutical composition of claim 6, wherein the *Prevotella stercorea* strain or a culture solution thereof increases the proliferation of CD4⁺ T cells and CD8⁺ T cells.

13. The pharmaceutical composition of claim 6, wherein the *Prevotella stercorea* strain or a culture solution thereof increases the interferon-γ (IFN-γ) secretion of T cells.

14. The pharmaceutical composition of claim 6, wherein the cancer is one or more selected from the group consisting of breast cancer, colorectal cancer, lung cancer, small cell lung cancer, gastric cancer, liver cancer, hematologic malignancy, bone cancer, pancreatic cancer, skin cancer, head or neck cancer, cutaneous or intraocular melanoma, uterine cancer, ovarian cancer, rectal cancer, cancer near the anus, colon cancer, fallopian tube carcinoma, endometrial carcinoma, cervical cancer, vaginal cancer, vulva carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphotic lymphoma, bladder cancer, renal cancer, ureter cancer, renal cell carcinoma, renal pelvis carcinoma, CNS tumors, primary CNS lymphoma, spinal cord tumors, brainstem glioma and pituitary adenoma.

15. A method for enhancing immunity or increasing an anticancer effect, the method comprising administering a composition comprising a *Prevotella stercorea* strain or a culture solution thereof to a subject in need thereof.

16. A use of a composition comprising a *Prevotella stercorea* strain or a culture solution thereof for enhancing immunity or increasing an anticancer effect.

17. A use of a *Prevotella stercorea* strain or a culture solution thereof for producing an immune enhancer or an agent for increasing an anticancer effect.

18. A method for preventing or treating cancer, the method comprising administering a pharmaceutical composition comprising a *Prevotella stercorea* strain or a culture solution thereof; and an immune checkpoint inhibitor as an active ingredients to a subject in need thereof.

19. A use of a pharmaceutical composition comprising a *Prevotella stercorea* strain or a culture solution thereof; and an immune checkpoint inhibitor as an active ingredients for preventing or treating cancer.

20. A use of a *Prevotella stercorea* strain or a culture solution thereof; and an immune checkpoint inhibitor for producing a therapeutic agent for cancer.
